# EUROPÄISCHE PATENTSCHRIFT

(11) **EP 0 623 591 B1**
(45) Veröffentlichungstag und Bekanntmachung des Hinweises auf die Patenterteilung: **25.09.1996**
(21) Anmeldenummer: 94105985.9
(22) Anmeldetag: 18.04.1994
(51) Int. Cl.: C07C 265/04, C07C 265/10, C08G 18/78, C08G 18/80

(54) **Olefinisch ungesättigte Isocyanate und ihre Verwendung als Bindemittel in Beschichtungsmaterialien**
Olefinically unsaturated isocyanates and their use as binder in coating materials
Dérivés d'isocyanates oléfiniquement insaturés et leur application comme liants dans des produits de revêtement

(30) Priorität: 30.04.1993 DE 4314252
(43) Veröffentlichungstag der Anmeldung: 09.11.1994
(73) Patentinhaber: BAYER AG, 51368 Leverkusen (DE)
(72) Erfinder: Brahm, Martin, Dr., D-51766 Engelskirchen (DE); Arning, Eberhard, Dipl.-Ing., D-41564 Kaarst (DE); Schmalstieg, Lutz, Dr., D-50676 Köln (DE); Schwindt, Jürgen, Dr., D-51373 Leverkusen (DE); Pedain, Josef, Dr., D-51061 Köln (DE)

(56) Entgegenhaltungen:
- EP-A- 0 301 345
- EP-A- 0 451 657
- DE-A- 2 452 074
- DE-B- 1 230 778
- FR-A- 2 255 294

## Beschreibung

Die Erfindung betrifft ein Verfahren zur Herstellung von olefinisch ungesättigten Isocyanaten durch Umsetzung von a1) 1-Isocyanato-3,3,5-trimethyl-5-isocyanato-methyl-cyclohexan (Isophorondiisocyanat, abgekürzt: IPDI) oder von Gemischen von IPDI mit a2) anderen organischen Polyisocyanaten mit b1.1) ausgewählten olefinisch ungesättigten, vorzugsweise einwertigen Carbonsäuren oder Gemischen derartiger Carbonsäuren mit b1.2) anderen organischen Carbonsäuren, b2.1) olefinisch ungesättigten Alkoholen und/oder b2.2) gesättigten Alkoholen, die so erhältlichen olefinisch ungesättigten Isocyanate und ihre Verwendung als Bindemittel in Beschichtungsmaterialien.

Einkomponentig zu verarbeitende Beschichtungsmittel auf Basis NCO-funktioneller Urethangruppen enthaltender Prepolymere sind seit langem bekannt (Houben-Weyl, Methoden der organischen Chemie, Band E20, Seite 1646, Georg Thieme Verlag 1987). Sie werden hergestellt durch Reaktion von Diisocyanaten bzw. modifizierten Diisocyanaten mit höherfunktionellen Polyolen wie z.B. Polyether- und Polyesterpolyolen. Der höhermolekulare Aufbau verleiht diesen Verbindungen gute filmbildende und filmoptische Eigenschaften, jedoch ebenso eine höhere Viskosität weshalb sie nur stark verdünnt oder mit erheblichem Anteil an monomerem Diisocyanat als Lackbindemittel bzw. als Beschichtungsmaterial eingesetzt werden können. Hohe Konzentrationen an monomerem Isocyanat sind jedoch aus physiologischer Sicht nicht akzeptabel. Darüber hinaus ist der Einsatz von großen Mengen an Lösungsmittel umweltpolitisch nicht vertretbar. Die Schere zwischen einem hochmolekularen, hochfunktionellen Aufbau mit hoher Viskosität aber positiven Produkteigenschaften einerseits und niedermolekularen niedrigviskosen Produkten aber ungenügender Lösungsbeständigkeit und ungenügenden Trocknungseigenschaften andererseits läßt sich durch NCO-funktionelle Prepolymere nach dem oben erwähnten Aufbauprinzip nicht schließen.

Neben NCO-funktionellen Beschichtungsmaterialien sind auch oxidativ vernetzbare 1 K-Systeme beschrieben (Ullmann, Encyclopädie der technischen Chemie, 4. Auflage, Band 19, Seite 75 ff., Verlag Chemie Weinheim, Deerfield Beach Florida, Basel 1980). Diese sind ebenfalls nur in verdünnten Lösungen als Bindemittel für Beschichtungsmaterialien einsetzbar.

Es war daher die der Erfindung zugrundeliegende Aufgabe, neue, niedrigviskose Bindermittel für lösungsmittelfreie bzw. -arme Beschichtungsmaterialien mit guten lacktechnischen Eigenschaften, schneller chemischer Trocknung bei Raumtemperatur und universeller und physiologisch unbedenklicher Anwendbarkeit zur Verfügung zu stellen.

Diese Aufgabe konnte mit dem nachstehend näher beschriebenen erfindungsgemäßen Verfahren gelöst werden, welches zu Verfahrensprodukten führt, die den genannten Anforderungen wegen des gleichzeitigen Vorliegens von olefinischen Doppelbindungen, Amid- und gegebenenfalls Urethangruppen, sowie Isocyanatgruppen genügen. Wesentlich ist hierbei insbesondere ein ausgewogenes Verhältnis von Amidgruppen, Isocyanatgruppen und zur oxidativen Vernetzung befähigen Doppelbindungen.

Gegenstand der Erfindung ist ein Verfahren zur Herstellung von olefinisch ungesättigten Isocyanaten mit einem NCO-Gehalt von 4 bis 20 Gew.-%, dadurch gekennzeichnet, daß man
a) eine Isocyanatkomponente mit einen NCO-Gehalt von 20 bis 51 Gew.-% und einer (mittleren) Funktionalität von unter 2,5, bestehend im wesentlichen ans
   a1) 40 bis 100 Gew.-% Isophorondiisocyanat (IPDI),
   a2) 0 bis 60 Gew.-% an anderen organischen Polyisocyanaten mit einem NCO-Gehalt von 10 bis 60 Gew.-%
   mit
b) einer olefinisch ungesättigten Reaktivkomponente einer (mittleren) Funktionalität im Sinne der Isocyanat-Additionsreaktion von unter 1,5 und einer Iodzahl von über 70, bestehend im wesentlichen aus
   b1) 1 bis 100 Gew.-% einer Carbonsäurekomponente aus
      b1.1) 80 bis 100 Gew.-% mindestens einer einwertigen, olefinisch ungesättigten Carbonsäure mit 10 bis 22 Kohlenstoffatomen pro Molekül und
      b1.2) 0 bis 20 Gew.-% an anderen ein- oder mehrwertigen Carbonsäuren des Molekulargewichtsbereichs 46 bis 350, und
   b2) 0 bis 99 Gew.-% an einer Alkoholkomponente aus
      b2.1) 0 bis 100 Gew.-% mindestens eines einwertigen olefinisch ungestättigten Alkohols mit 10 bis 22 Kohlenstoffatomen pro Molekül und
      b2.2) 0 bis 100 Gew.-% anderen ein- oder mehrwertigen Alkoholen des Molekulargewichtsbereichs 32 bis 4000, mit der Maßgabe, daß die Gesamtmenge der Alkohole b2.2) bei maximal 20 Gew.-%, bezogen auf das Gesamtgewicht der Komponente b) liegt,
gegebenenfalls in Anwesenheit von Katalysatoren und/oder anderen Hilfs- und Zusatzmitteln der Isocyanatchemie unter Einhaltung eines Äquivalentverhältnisses von Isocyanatgruppen der Komponente a) zu Carboxyl- und gegebenenfalls Hydroxylgruppen der Komponente b) von 4:1 bis 40:1 umsetzt und anschließend die Umsetzungsprodukte destillativ von überschüssigen, destillierbaren Ausgangsisocyanaten a1) und/oder a2) bis zu einem Restgehalt an solchen Isocyanaten von maximal 0,5 Gew.-% befreit.

Gegenstand der Erfindung sind auch die nach diesem Verfahren erhältlichen, olefinisch ungesättigten Isocyanate und ihre Verwendung als Bindemittel für bei Raumtemperatur einkomponentig zu verarbeitende Beschichtungsmaterialien.

Die Umsetzung von organischen Carbonsäuren mit überschüssigen Mengen an organischen Diisocyanaten ist aus der DE-AS 1 230 778 bereits bekannt. Bei den konkret beschriebenen Verfahrensprodukten dieser Vorveröffentlichung handelt es sich jedoch um acylierte Harnstoffpolyisocyanate, die für die üblichen Einsatzgebeite der Polyurethanchemie Verwendung finden können. Die Herstellung von Amidgruppen aufweisenden olefinisch ungesättigten Isocyanaten der Art der nachstehend näher beschriebenen erfindungsgemäßen Verfahrensprodukte wird ebensowenig beschrieben wie ihre Verwendung als Bindemittel für einkomponentig zu verarbeitende Beschichtungsmaterialien.

Dem erfindungsgemäßen Verfahren liegt die überraschende Beobachtung zugrunde, daß bei Verwendung von Isophorondiisocyanat oder von im wesentlichen aus Isophorondiisocyanat bestehenden Polyisocyanatgemischen als Reaktionspartner für Carbonsäuren im Unterschied zur Lehre der DE-AS 1 230 778 keine bzw. nur in untergeordnetem Maße Acylharnstoffgruppen aufweisende Verfahrensprodukte entstehen. Bei den erfindungsgemäßen Verfahrensprodukten handelt es sich vielmehr um im wesentlichen Amidgruppen enthaltende Verbindungen, was sich beispielsweise NMR-spektroskopisch nachweisen läßt. Die im wesentlichen Acylharnstoff-freien Verfahrensprodukte sind kristallisationsstabil und niedrigviskos. Ein wesentlicher Vorteil liegt außerdem in den schnellen Trocknungszeiten bei ihrer Verwendung als Lackbindemittel was auf das ausgewogene Verhältnis von Isocyanatgruppen, Amidgruppen und zur oxidativen Vernetzung befähigten Doppelbindungen zurückzuführen ist.

Die erfindungsgemäßen Verfahrensprodukte weisen einen NCO-Gehalt von 20 bis 51, vorzugsweise 6 bis 13 Gew.-%, eine Iodzahl von 20 bis 250, vorzugsweise 30 bis 150 und einen Gehalt an destillierbaren Ausgangsisocyanaten von unter 0,5, vorzugsweise von unter 0,2 Gew.-% auf. Ihre Viskosität bei 23°C liegt lösungsmittelfrei im allgemeinen unter 30.000 mPa.s.

Bei der Isocyanatkomponente a) handelt es sich um a1) Isophorondiisocyanat oder um Gemische von Isophorondiisocyanat mit a2) anderen organischen Polyisocyanaten, wobei diese, falls überhaupt, in Mengen von bis zu 60, vorzugsweise bis zu 40 und ganz besonders bevorzugt bis zu 30 Gew.-% bezogen auf das Gesamtgewicht der Komponente a) zum Einsatz gelangen.

Ausgangsisocyanate a2) sind solche mit einem NCO-Gehalt von 10 bis 60 Gew.-%. In Betracht kommen beispielsweise aliphatische und cycloaliphatische Diisocyanate wie 1,4-Diisocyanatobutan, 1,5-Diisocyanatopentan, 1,6-Diisocyanatohexan (HDI), Dodecamethylendiisocyanat, Undecandiisocyanat und 2,2,4-Trimethylhexandiisocyanat, 1,3-Cyclopentylendiisocyanat, Cyclohexan-1,3- und 1,4-diisocyanat, die Isomeren Diisocyanatodicyclohexylmethane, 2,5- und 2,6-Bis(isocyanatomethyl)bicyclo[2.2.1]heptan, Isocyanatomethyl-1-methylcyclohexylisocyanat (IMCI), 1,4- und 1,3-Di(isocyanatoisopropyl)cyclohexan, Xylylendiisocyanat und Diisocyanate mit aromatisch gebundenen Isocyanatgruppen wie 2,4-Diisocyanatotoluol, 2,6-Diisocyanatotoluol, aus diesen Isomeren bestehende Gemische, isomere Diisocyanatodiphenylmethane und höhere Homologe, 1,3-Phenylendiisocyanat, 1,4-Phenylendiisocyanat, 4,4'-Biphenylendiisocyanat, 1,5-Naphthylendiisocyanat, 1,4-Naphthylendiisocyanat und 4,4'-Diisocyanatodiphenylether sowie deren Gemische.

Unter den Ausgangsisocyanaten a2) sind auch Modifizierungsprodukte der oben erwähnten Diisocyanate mit Biuret-, Uretdion-, Isocyanurat-, Allophanat- und Carbodiimidgruppen zu verstehen. Es ist auch möglich zur Einstellung spezieller Eigenschaften monofunktionelle Isocyanate zu verwenden. Dies ist jedoch keineswegs bevorzugt.

Ganz besonders bevorzugt wird als Isocyanatkomponente a) ein Gemisch aus 70 bis 100 Gew.-% Isophorondiisocyanat und 0 bis 30 Gew.-% 1,6-Diisocyanatohexan eingesetzt.

Die Reaktivkomponente b) weist eine (mittlere) Funktionalität im Sinne der Isocyanat-Additionsreaktion von 1 bis 1,5, vorzugsweise von 1 auf. Ihre Iodzahl liegt über 90, vorzugsweise bei 100 bis 400. Die Reaktivkomponente b) besteht zu 1 bis 100, vorzugsweise 80 bis 100 und besonders bevorzugt zu 100 Gew.-% aus einer Carbonsäurekomponente b1) und zum Rest aus einer Alkoholkomponente b2).

Die Carbonsäurekomponente b1) besteht ihrerseits zu 80 bis 100 Gew.-%, vorzugsweise 100 Gew.-% aus olefinisch ungesättigten Monocarbonsäuren b1.1) und zum Rest aus anderen Mono- oder Polycarbonsäuren b1.2).

Die Alkoholkomponente b2) besteht ihrerseits zu 0 bis 100, vorzugsweise 80 bis 100 Gew.-% aus einwertigen, olefinisch ungesättigten Alkoholen b2.1) und zum Rest aus anderen ein- oder mehrwertigen Alkoholen b2.2), wobei die Gesamtmenge der Alkoholkomponente b2.2), bezogen auf das Gesamtgewicht der Komponente b) bei maximal 20, vorzugsweise maximal 10 Gew.-% liegt.

Geeignete Carbonsäuren b1.1) sind olefinisch ungesättigte Monocarbonsäuren mit (im Mittel) 10 bis 22, vorzugsweise 14 bis 20 Kohlenstoffatomen pro Molekül und einer Iodzahl von über 70, vorzugsweise über 90 und besonders bevorzugt 100 bis 400. Gut geeignet sind beispielsweise Monocarbonsäuren bzw. Gemische, die sich von den entsprechenden ungesättigten synthetischen oder natürlichen Fettsäuren ableiten. Beispielhaft genannt seien die isomeren Decensäuren, Dodecensäuren, Tetradecensäuren, Hexadecensäuren, Octadecensäuren, Eicosensäuren, Docosensäuren, Tetracosensäuren, Ximensäuren, 12-Oxyoctadecensäuren, Octadecadiensäuren, Octadecatriensäuren, Ecosatetraensäuren, Docosapentaensäuren oder auch Rizinolsäure. Natürlich vorkommende Fettsäuregemische sind beispielsweise die aus Rizinusöl, Erdnußölfett, Baumwollsaatöl, Safloröl, Holzöl, Sajaöl, Sonnenblumenöl, Leinöl, Rüböl, Talöl, Spermöl und Heringsöl abgeleiteten Säuren. Grundsätzlich möglich, jedoch nicht bevorzugt, ist auch die Verwendung von olefinisch ungesättigten Hydroxycarbonsäuren als Komponente b1.1). Falls derartige Säuren eingesetzt werden, erfüllen sie die Doppelfunktion einer ungesättigten Carbonsäure b1.1) und eines ungesättigten Alkohols b2.1). Bei der Berechnung der Mengen-bzw. Äquivalentverhältnisse der Einzelkomponenten der Komponente b) muß daher diesem Umstand entsprechend Rechnung getragen werden.

Bei den gegebenenfalls mitverwendeten anderen Carbonsäuren b1.2) handelt es sich um solche des Molekulargewichtes 46 bis 600, vorzugsweise 60 bis 300. Es kommen sowohl einwertige als auch mehrwertige Carbonsäuren in Betracht. Beispielhaft genannt seien Ameisensäure, Essigsäure, die isomeren Propansäuren, Butansäuren, Pentansäuren, Hexansäuren, Heptansäuren, Octansäuren, Nonansäuren, Decansäuren, Dodecansäuren, Tetradecansäuren, Hexadecansäuren, Octadecansäuren, Eicosansäuren, Docosansäuren, Tetracosansäuren, Dicarbonsäuren wie Maleinsäure, Fumarsäure, Malonsäure, Adipinsäure, Sebazinsäure und Dimerprodukte der ungesättigten Fettsäuren b1) Tricarbonsäuren wie Trimilitsäure, Citronensäure und Trimerprodukte der ungesättigten Fettsäuren b1), Tetracarbonsäuren wie Benzoltetracarbonsäure, bzw. Gemische beliebiger Carbonsäuren.

Geeignete Alkohole b2.1) sind olefinisch ungesättigte Alkohole oder Gemische von olefinisch ungesättigten Alkoholen mit (im Mittel) 10 bis 22, vorzugsweise 14 bis 20 Kohlenstoffatomen pro Molekül und Iodzahlen von über 70, vorzugsweise von über 90 und besonders bevorzugt von 100 bis 400. Gut geeignet sind beispielsweise einwertige Alkohole bzw. Alkoholgemische, die sich von den entsprechenden ungesättigten synthetischen oder natürlichen Fettsäuren bzw. Fettsäuregemischen ableiten. Beispielhaft genannt seien die durch Reduktion von ungesättigten Carbonsäuren der vorstehend unter b1.1) genannten Art erhaltenen Alkohole.

Bei den gegebenenfalls mitverwendeten anderen Alkoholen b2.2) handelt es sich um solche des Molekulargewichtsbereich 32 bis 4000, vorzugsweise 74 bis 286. Es kommen sowohl gesättigte einwertige als auch mehrwertige Alkohole in Betracht. Beispielhaft genannt seien Methanol Ethanol, n-Propanol, Isopropanol, die isomeren Butanole, Pentanole oder Hexanole, n-Octanol n-Dodecanol oder n-Octadecanol, gesättigte Fettalkohole oder mehrwertige Alkohole wie Ethylenglykol, Propylenglykol, die isomeren Butandiole, Hexandiole oder Octandiole, Glyzerin, Trimethylolpropan, oder auch Polymerpolyole wie beispielsweise Polyether-Polycarbonat- oder Polyacrylatpolyole des genannten Molekulargewichtsbereichs. Gemische derartiger Alkohole können ebenfalls eingesetzt werden. Die Carbonsäuren b1.2) und die Alkohole b2.2) werden, falls überhaupt, in solchen Mengen innerhalb der genannten Bereiche eingesetzt, daß die genannten Bedingungen bezüglich Funktionalität und Iodzahl der Komponente b) erfüllt sind.

Zur Durchführung des erfindungsgemäßen Verfahrens werden die Komponenten a) und b) in solchen Mengen miteinander zur Reaktion gebracht, die einem Äquivalentverhältnis von Isocyanatgruppen der Komponente a) zu Carboxyl- und gegebenenfalls Hydroxylgruppen der Komponente b) von 4:1 bis 40:1, vorzugsweise 5:1 bis 20:1 und besonders bevorzugt 6:1 bis 15:1 entsprechen. Die Umsetzung erfolgt im allgemeinen innerhalb des Temperaturbereiches von 80 bis 200°C, vorzugsweise von 100 bis 170°C, wobei zur Beschleunigung gegebenenfalls übliche Katalysatoren wie beispielsweise Triethylamin, Tributylamin, N,N,N',N'-Tetramethylbutyl-1,4-diamin, Bis(dimethylamino)ethylether, Dimethylethanolamin, 1,4-Diazabicyclo-[2.2.2]-octan, Diazabicycloundecen, N,N-Dimethylbenzylamin 1- und 2-Methylimidazol, Tris(dimethylaminomethyl)phenol, Pyridin, Mannichbasen, Morpholine, Tetraalkylammoniumhydroxide und Alkalihydroxide wie Natriumhydroxid, Alkaliphenolate, Metallsalze wie Eisen-(III)-chlorid, Kaliumoctoat, Zinnverbindungen wie Zinn-(II)-octoat, Zinn-(II)-ethylhexanoat, Zinn-(II)-laurat, Aluminium-tri(ethylacetoacetat), Dibutylzinnoxid, Dibutylzinndichlorid, Dibutylzinndiacetat, Dibutylzinndilaurat, Dibutylzinnmaleat oder Dioctylzinndiacetat und Mineralsäuren wie Schwefelsäure, Salzsäure, Phosphorsäure und Perchlorsäure eingesetzt werden können. Werden Alkohole b2) mit eingesetzt, kann es sinnvoll sein diese in einer Vorreaktion im Temperaturbereich von 30 bis 100°C mit der Isocyanatkomponente zur Reaktion zu bringen und anschließend die Carbonsäurekomponente b1) mit dem Vorprodukt weiter umzusetzen.

Im Anschluß an die Umsetzung wird das überschüssige, destillierbare Ausgangsdiisocyanat destillativ, vorzugsweise durch Dünnschichtdestillation bis auf einen Restgehalt im Verfahrensprodukt von unter 0,5, vorzugsweise unter 0,2 Gew.-% entfernt.

Bei den erfindungsgemäßen Verfahrensprodukten handelt es sich um wertvolle, unter dem Einfluß von Luftfeuchtigkeit und Luftsauerstoff aushärtbare Bindemittel für Beschichtungsmaterialien.

Vor der erfindungsgemäßen Verwendung der erfindungsgemäßen Verfahrensprodukte werden diesen im allgemeinen an sich bekannte, die oxidative Vernetzung beschleunigende Katalysatoren zugesetzt. Derartige Katalysatoren sind beispielsweise in Ullmann, Enzyklopädie der technischen Chemie, 4. Auflage, Band 23, Seite 42 (Trockenstoffe) Verlag Chemie 1983, sowie in der Offenlegungsschrift DE 4 032 546 und darin zitierten Schriften aufgeführt. Als Beispiele seien Kobalt-, Blei-, Magnesium-, Zirkonium-, Aluminium-, Mangan-, Calcium-, Cer-, Kupfer-, Nickel-, Vanadium-, Barium- und Zinksikkative sowie Gemische genannt.

Auch Katalysatoren zur Beschleunigung der Isocyanat-Additionsreaktion der oben beispielhaft genannten Art können den erfindungsgemäßen Verfahrensprodukten vor ihrer erfindungsgemäßen Verwendung zugesetzt werden.

Zur Erzeugung eines speziellen Eigenschaftsprofils der Beschichtungsmassen kann es sinnvoll sein, andere nichtfunktionelle Polymere bzw. NCO-funktionelle Zusätze und Polymerkomponenten, die zur oxidativen Vernetzung befähigt sind, als weitere Bindemittelkomponenten mitzuverwenden. Derartige weitere Bindemittelkomponenten werden im allgemeinen in einer Menge von maximal 30, vorzugsweise maximal 10 Gew.-% mitverwendet. Ganz besonders bevorzugt wird auf die Mitverwendung weiterer Bindemittelkomponenten bei der erfindungsgemäßen Verwendung der erfindungsgemäßen Verfahrensprodukte verzichtet.

Als weitere Bindemittelkomponenten kommen beispielsweise Alkydharze in Betracht wie sie z.B. in Römpps Chemielexikon, Band 1, Seite 202, Frankh'sche Verlagsbuchhandlung, Stuttgart, 1966 definiert oder bei D.H. Solomon, The Chemistry of Organic Filmformers, S. 75 bis 101, John Wiley / Sons Inc., New York 1967, beschrieben sind. NCO-funktionelle Bindermittelkomponenten, die neben den erfindungsgemäßen Verfahrensprodukten eingesetzt werden können sind beispielsweise die bekannten Lackpolyisocyanate, d.h. vorzugsweise (i) Urethangruppen, (ii) Isocyanurat- und/oder Uretdiongruppen oder (iii) Biuretgruppen aufweisende Derivate von aliphatischen Diisocyanaten, insbesondere von 1,6-Diisocyanatohexan.

Neben den erfindungsgemäßen Verfahrensprodukten und den beispielhaft genannten, gegebenenfalls mitverwendeten weiteren Bindemittelkomponenten können in den Beschichtungsmitteln auch andere Hilfs- und Zusatzmittel mitverwendet werden. Hierzu gehören beispielsweise Lösungsmittel, die allerdings nur in geringen Mengen mitverwendet werden, so daß der Feststoffanteil der Beschichtungsmaterialien über 85, vorzugsweise über 90 Gew.-% liegt, vorzugsweise wird auf die Mitverwendung von Lösungsmitteln verzichtet. Geeignete Lösungsmittel sind beispielsweise Toluol, Xylol, Cyclohexan, Chlorbenzol, Butylacetat, Ethylacetat, Ethylglykolacetat, Pentylacetat, Hexylacetat, Methoxypropylacetat, Tetrahydrofuran, Dioxan, Aceton, Methylethylketon, Xylol, Testbenzin, höhere Aromaten, wie sie beispielsweise unter der Bezeichnung ®Solvesso oder ®Shellsol im Handel erhältich sind, oder beliebige Gemische derartiger Lösungsmittel. Besonders gut geeignet sind geruchsarme isoparaffinische Lösungsmittel, wie sie beispielsweise unter den Bezeichnungen ®Isopar oder ®Nappar im Handel erhältich sind. Die erfindungsgemäßen Verfahrensprodukte weisen eine gute Verträglichkeit und Mischbarkeit mit diesen unpolaren Lösungsmitteln auf.

Als weitere Hilfsstoffe können in den erfindungsgemäßen Beschichtungsmaterialien auch beispielsweise die üblichen Benetzungsmittel, Verlaufsmittel, Hautverhinderungsmittel, Antischaummittel Mattierungsmittel wie beispielsweise Kieselsäure, Aluminiumsilikate und hochsiedende Wachse, viskositätsregulierende Stoffe, Pigmente, Farbstoffe, UV-Absorber oder Stabilisatoren gegen thermischen bzw. oxidativen Abbau eingesetzt werden.

Die der erfindungsgemäßen Verfahrensprodukte als wesentliche Bindemittel enthaltende Beschichtungsmaterialien können zur Beschichtung beliebiger Substrate wie beispielsweise Holz, Kunststoff, Leder, Papier, Textilien, Glas, Keramik, Putz, Mauerwerk, Metalle oder Beton verwendet werden. Sie lassen sich mit üblichen Applikationsmethoden wie Spritzen, Streichen, Fluten, Gießen, Tauchen, Walzen aufbringen. Die Beschichtungsmittel können in form von Klarlacken als auch in Form pigmentierter Lacke verwendet werden.

Die so hergestellten Beschichtungen härten bei 20°C im allgemeinen während eines Zeitraums von 2 bis 24 Stunden zu hochwertigen Überzügen aus. Die Härtung kann jedoch auch bei tieferen Temperaturen (bis -5°C) oder beschleunigt bei höheren Temperaturen (bis beispielsweise 130°C) erfolgen.

In den nachfolgenden Beispielen beziehen sich alle Angaben in "Teilen" und in "%" auf das Gewicht.

### Beispiele 1 bis 3 (Herstellung und Eigenschaften der erfindungsgemäßen Produkte)

In einer mit Stickstoff gespülten Rührapparatur werden jeweils x val Isocyanatkomponente vorgelegt und bei 150°C mit y val entwässerten Carbonsäurekomponente tropfenweise innerhalb von 3 bis 4 Stunden versetzt. Nach 6 bis 7 Stunden Reaktionsdauer unter Stickstoffatmosphäre läßt man abkühlen. Nachfolgend wird das überschlüssige Diisocyanat durch Dünnschichtdestillation im Hochvakuum (0,1 bis 0,3 mbar) bei einer Temperatur von 150°C abgetrennt. In Tabelle 1 sind die Produktdaten zusammengefaßt.

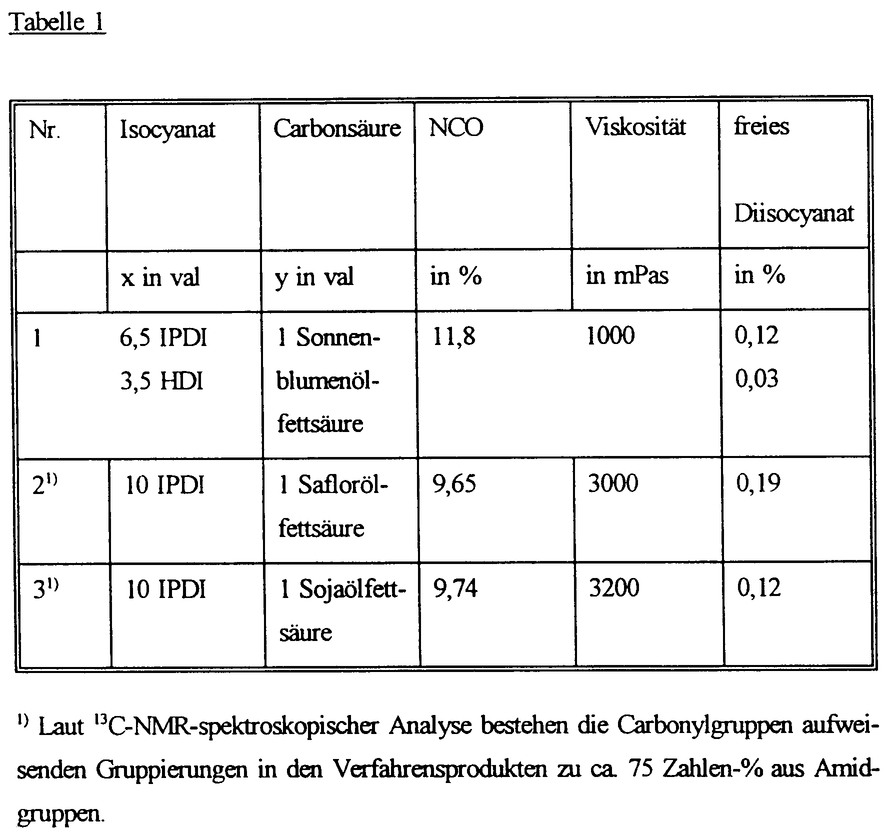

### Vergleichsbeispiele 4 bis 7 (Herstellung und Eigenschaften der nicht erfindungsgemäßen Vergleichsprodukte, hergestellt wie under Beispiel 1 siehe Tabelle 1)

**Tabelle 2**

| Nr. | Isocyanat | Carbonsäure | NCO | Viskosität | freies Diisocyanat |
|---|---|---|---|---|---|
| | in val | in val | in % | in mPas | in % |
| 4 | 10 HDI | 1 Sonnenblumenölfettsäure | - | kristallin | - |
| 5 | 10 HDI | 1 Sojaölfettsäure | - | kristallin | - |
| 6 | 2 IPDI | 1 Sojaölfettsäure | 12,5 | zum Teil kristallin | 0,18 |
| | 8 HDI | | | | 0,03 |
| 7* | 1 IPDI | 1 Sojaölfettsäure | <1 | 92000 | - |

| | | | | | |
|---|---|---|---|---|---|
| * nicht dünnschichtdestilliertes Produkt | | | | | |

### Beispiele 8 bis 10 (Herstellung und Eigenschaften von erfindungsgemäßen Klarlackfilmen)

Eine Basisformulierung für Klarlacke hat die folgende Zusammensetzung:
93,4 Teile Lackbindemittel
2,8 Teile Octa Soligen Calcium 4
0,5 Teile Octa Soligen Kobalt 6
2,8 Teile Octa Soligen Zirkon 18
0,5 Teile Ascinin R konz. (handelsübliches Hautverhinderungsmittel)

Es werden auf gereinigte Glasplatten in einer Schichtdicke von 120 µm die folgenden Bindemittel-Formulierungen aufgetragen und bei Raumtemperatur ausgehärtet. Die resultierenden Trocknungszeiten (Sandtrocknung nach Stunden bei 20°C), Druckfestigkeiten (nach DIN 53 150), Werte für die Pendeldämpfung (nach DIN 53 157) und die Lösungsmittelbeständigkeit (0 = unverändert, 5 = aufgelöst) werden in nachfolgender Tabelle 3 zusammengefaßt.

**Tabelle 3**

| Nr. | Bindemittel aus Beispiel | Sandtrocknung nach Stunden | druckfest nach Stunden | Pendeldämpfung nach 7d in s | Lösungsmittelfestigkeit |
|---|---|---|---|---|---|
| 8 | 1 | 3 | >8<18 | 75 | 1-2 |
| 9 | 2 | 2,5 | >8<18 | 112 | 1-2 |
| 10 | 3 | 4,5 | >8<18 | 110 | 1-2 |

### Vergleichsbeispiele 11 und 12 (nicht erfindungsgemäß):

Bei gleicher Basisformulierung wie unter Beispiel 8 bis 10 zeigen die Vergleichsbeispiele 11 und 12, die auf den Bindemitteln gemäß Vergleichsbeispielen 6 und 7 basieren, die in Tabelle 4 aufgeführten Trocknungseigenschaften:

**Tabelle 4**

| Nr. | Bindemittel aus Vergleichsbeispiel | Sandtrocknung in Stunden | druckfest nach Stunden | Pendeldämpfung nach 7d in s |
|---|---|---|---|---|
| 11 | 6 | >8 | >30 | 80 |
| 12 | 7 | >8 | >30 | 63 |

## Patentansprüche

1. Verfahren zur Herstellung von olefinisch ungesättigten Isocyanaten mit einem NCO-Gehalt von 4 bis 20 Gew.-%, dadurch gekennzeichnet, daß man
a) eine Isocyanatkomponente mit einen NCO-Gehalt von 20 bis 51 Gew.-% und einer (mittleren) Funktionalität von unter 2,5, bestehend im wesentlichen aus
a1) 40 bis 100 Gew.-% Isophorondiisocyanat (IPDI),
a2) 0 bis 60 Gew.-% an anderen organischen Polyisocyanaten mit einem NCO-Gehalt von 10 bis 60 Gew.-%
mit
b) einer olefinisch ungesättigten Reaktivkomponente einer (mittleren) Funktionalität im Sinne der Isocyanat-Additionsreaktion von unter 1,5 und einer Iodzahl von über 70, bestehend im wesentlichen aus
b1) 1 bis 100 Gew.-% einer Carbonsäurekomponente aus
b1.1) 80 bis 100 Gew.-% mindestens einer einwertigen, olefinisch ungesättigten Carbonsäure mit 10 bis 22 Kohlenstoffatomen pro Molekül und
b1.2) 0 bis 20 Gew.-% an anderen ein- oder mehrwertigen Carbonsäuren des Molekulargewichtsbereichs 46 bis 350, und
b2) 0 bis 99 Gew.-% an einer Alkoholkomponente aus
b2.1) 0 bis 100 Gew.-% mindestens eines einwertigen olefinisch ungestättigten Alkohols mit 10 bis 22 Kohlenstoffatomen pro Molekül und
b2.2) 0 bis 100 Gew.-% anderen ein- oder mehrwertigen Alkoholen des Molekulargewichtsbereichs 32 bis 4000, mit der Maßgabe, daß die Gesamtmenge der Alkohole b2.2) bei maximal 20 Gew.-%, bezogen auf das Gesamtgewicht der Komponente b) liegt,
gegebenenfalls in Anwesenheit von Katalysatoren und/oder anderen Hilfs- und Zusatzmitteln der Isocyanatchemie unter Einhaltung eines Äquivalentverhältnisses von Isocyanatgruppen der Komponente a) zu Carboxyl- und gegebenenfalls Hydroxylgruppen der Komponente b) von 4:1 bis 40:1 umsetzt und anschließend die Umsetzungsprodukte destillativ von überschüssigen, destillierbaren Ausgangsisocyanaten a1) und/oder a2) bis zu einem Restgehalt an solchen Isocyanaten von maximal 0,5 Gew.-% befreit.

2. Verfahren gemäß Anspruch 1, dadurch gekennzeichnet, daß die Komponente a) zu mindestens 60 Gew.-% aus Isophorondiisocyanat besteht.

3. Verfahren gemäß Anspruch 1 und 2, dadurch gekennzeichnet, daß die Komponente b) zu mindestens 80 Gew.-% aus Carbonsäuren b1) besteht.

4. Verfahren gemäß Anspruch 1 bis 3, dadurch gekennzeichnet, daß die Komponente b) ausschließlich ans ungesättigten Carbonsäuren b1.1) besteht.

5. Olefinisch ungesättigte Isocyanate, erhältlich nach dem Verfahren gemäß Anspruch 1 bis 4.

6. Verwendung der gemäß Anspruch 1 bis 4 erhältlichen olefinisch ungesättigten Isocyanate als Bindermittel für bei Raumtemperatur einkomponentig zu verarbeitende Beschichtungsmaterialien.

## Claims

1. A method for the preparation of olefinically unsaturated isocyanates having an isocyanate content of from 4 to 20 wt.%, characterised in that
a) an isocyanate component having an isocyanate content of from 20 to 51 wt.% and an (average) functionality of below 2.5, consisting substantially of
a1) from 40 to 100 wt.% of isophorone diisocyanate (IPDI),
a2) from 0 to 60 wt.% of other organic polyisocyanates having an isocyanate content of from 10 to 60 wt.%
is reacted with
b) an olefinically unsaturated reactive component having an (average) functionality in the isocyanate addition reaction of below 1.5 and an iodine number of above 70, consisting substantially of
b1) from 1 to 100 wt.% of a carboxylic acid component consisting of
b1.1) from 80 to 100 wt.% of at least one monobasic, olefinically unsaturated carboxylic acid having 10 to 22 carbon atoms per molecule and
b1.2) from 0 to 20 wt.% of other monobasic or polybasic carboxylic acids in the molecular weight range of from 46 to 350, and
b2) from 0 to 99 wt.% of an alcohol component consisting of
b2.1) from 0 to 100 wt.% of at least one monohydric olefinically unsaturated alcohol having 10 to 22 carbon atoms per molecule and
b2.2) from 0 to 100 wt.% of other monohydric or polyhydric alcohols in the molecular weight range of from 32 to 4000, with the proviso that the total quantity of the alcohols b2.2) is at most 20 wt.%, referred to the total weight of component b),
optionally in the presence of catalysts and/or other auxiliary substances and additives of isocyanate chemistry, with an equivalent ratio of isocyanate groups of component a) to carboxyl and optionally hydroxyl groups of component b) of from 4:1 to 40:1 being maintained, and the reaction products are subsequently freed by distillation from excess, distillable starting isocyanates a1) and/or a2) to a residual content of such isocyanates of at most 0.5 wt%.

2. The method according to claim 1, characterised in that component a) consists of isophorone diisocyanate to an extent of at least 60 wt.%.

3. The method according to claim 1 and 2, characterised in that component b) consists of carboxylic acids b1) to an extent of at least 80 wt.%.

4. The method according to claims 1 to 3, characterised in that component b) consists entirely of unsaturated carboxylic acids b1.1).

5. Olefinically unsaturated isocyanates, obtainable by the method according to claims 1 to 4.

6. Use of the olefinically unsaturated isocyanates obtainable according to claims 1 to 4 as binders for coating materials to be processed as one-component products at room temperature.

## Revendications

1. Procédé pour la préparation d'isocyanates à insaturation oléfinique possédant une teneur NCO de 4 à 20% en poids, caractérisé en ce qu'on fait réagir
a) un composant d'isocyanate possédant une teneur NCO de 20 à 51% en poids et une fonctionnalité (moyenne) inférieure à 2,5, constitué essentiellement par
a1) de 40 à 100%, en poids d'isophoronediisocyanate (IPDI),
a2) de 0 à 60% en poids d'autres polyisocyanates organiques possédant une teneur NCO de 10 à 60% en poids,
avec
b) un composant réactif à insaturation oléfinique possédant une fonctionnalité (moyenne), dans le sens de la réaction d'addition d'isocyanate, inférieure à 1,5 et un indice d'iode supérieur à 70, constitué essentiellement par
b1) de 1 à 100% en poids d'un composant d'acide carboxylique constitué par
b1.1) de 80 à 100% en poids d'au moins un acide carboxylique monovalent à insaturation oléfinique contenant de 10 à 22 atomes de carbone par molécule et
b1.2) de 0 à 20% en poids d'autres acides mono- ou polycarboxyliques du domaine de poids moléculaire de 46 à 350, et par
b2) de 0 à 99% en poids d'un composant d'alcool constitué par
b2.1) de 0 à 100% en poids d'au moins un alcool monovalent à insaturation oléfinique contenant de 10 à 22 atomes de carbone par molécule, et
b2.2) de 0 à 100% en poids d'autres alcools mono- ou polyvalents du domaine de poids moléculaire de 32 à 4.000, avec cette mesure que la quantité totale des alcools b2.2) se situe au maximum à 20% en poids rapportés au poids total du composant b),
éventuellement en présence de catalyseurs et/ou d'autres adjuvants et additifs de la chimie des isocyanates, tout en maintenant un rapport d'équivalents des groupes isocyanate du composant a) aux groupes carboxyle et éventuellement hydroxyle du composant b) de 4:1 à 40:1, et ensuite, on libère les produits réactionnels par distillation des isocyanates de départ en excès a1) et/ou a2) aptes à être distillés jusqu'à ce que l'on obtienne une teneur résiduelle en isocyanates de ce type de maximum 0,5% en poids.

2. Procédé selon la revendication 1, caractérisé en ce que le composant a) est constitué, à concurrence d'au moins 60% en poids, par l'isophoronediisocyanate.

3. Procédé selon les revendications 1 et 2, caractérisé en ce que le composant b) est constitué, à concurrence d'au moins 80% en poids, d'acides carboxyliques b1).

4. Procédé selon les revendications 1 à 3, caractérisé en ce que le composant b) est constitué exclusivement par des acides carboxyliques insaturés b1.1).

5. Isocyanates à insaturation oléfinique que l'on peut obtenir conformément au procédé selon les revendications 1 à 4.

6. Utilisation des isocyanates à insaturation oléfinique que l'on peut obtenir conformément aux revendications 1 à 4, comme liants pour des matières d'enduction à traiter comme matières à un seul composant à la température ambiante.
